# EUROPEAN PATENT SPECIFICATION

(11) **EP 1 546 325 B1**
(45) Date of publication and mention of the grant of the patent: **10.08.2011**
(21) Application number: 03795716.4
(22) Date of filing: 15.09.2003
(51) Int. Cl.: C12N 15/00

(54) **SYSTEMS OF SPERM CELL PRESERVATION AND PROCESSING**
SYSTEME ZUR ERHALTUNG UND VERARBEITUNG VON SPERMAZELLEN
SYSTEMES DE CONSERVATION ET DE TRAITEMENT DE CELLULES SPERMATIQUES

(30) Priority: 13.09.2002 US 410884 P; 09.01.2003 US 340881
(43) Date of publication of application: 29.06.2005
(60) Divisional application: 10186250.6
(73) Proprietor: XY, LLC, Navasota, TX 77868 (US)
(72) Inventor: MAXWELL, William, Maxwell, Chisholm, Beecroft, NSW 2119 (AU); HOLLINSHEAD, Fiona, Kate, Paddington, NSW 2021 (AU); O'BRIEN, Justine, Kellie, Paddington, NSW 2021 (AU); EVANS, Gareth, Lane Cove, NSW 2066 (AU)
(74) Representative: Kador, Utz Ulrich
(86) International application number: PCT/US2003/030814
(87) International publication number: WO 2004/024227

(56) References cited:
- WO-A-01/37655
- LU K H ET AL: "IN VITRO FERTILIZATION WITH FLOW-CYTOMETRICALLY-SORTED BOVINE SPERM" THERIOGENOLOGY, LOS ALTOS, CA, US, vol. 52, no. 8, December 1999 (1999-12), pages 1393-1405, XP000995618 ISSN: 0093-691X
- HOLLINSHEAD F K ET AL: "Sex-sorting and re-cryopreservation of frozen-thawed ram sperm for in vitro embryo production" THERIOGENOLOGY, LOS ALTOS, CA, US, vol. 59, no. 1, 1 January 2003 (2003-01-01), page 209, XP002981958 ISSN: 0093-691X
- HOLLINSHEAD F K ET AL: "Birth of lambs of a pre-determined sex after in vitro production of embryos using frozen-thawed sex-sorted and re-frozen-thawed ram spermatozoa." REPRODUCTION (CAMBRIDGE, ENGLAND) MAY 2004, vol. 127, no. 5, May 2004 (2004-05), pages 557-568, XP002427618 ISSN: 1470-1626
- SCHENK J.L. ET AL: 'Cryopreservation of Flow-Sorted Bovine Spermatozoa' THERIOGENOLOGY vol. 52, 1999, pages 1375 - 1391, XP002981957
- STAP J. ET AL: 'Improving the resolution of cryopreserved X- and Y-sperm during DNA flow cytometric analysis with the addition of percoll to quench the fluorescence of dead sperm' J. OF ANIMAL SCIENCE vol. 76, 1998, pages 1896 - 1902, XP000985715
- HOLLINSHEAD F.K.: 'In vitro and in vivo assessment of functional capacity of flow cytometrically sorted ram spermatozoa after freezing and thawing' REPROD. FERTIL. AND DEVELOP. vol. 15, 2003, pages 351 - 359, XP008037862
- HOLLINSHEAD F.K. ET AL: 'Sex-sorting and re-cryopreservation of frozen-thawed ram sperm for in vitro embryo production' THERIOGENOLOGY vol. 59, no. 1, 01 January 2003, page 209, XP002981958
- HOLLINSHEAD F.K. ET AL: 'Production of lambs of predetermined sex after the insemination of ewes with lownumbers of frozen-thawed sorted X- or Y-chromosome-bearing spermatozoa' REPROD. FERTIL. AND DEVLOP. vol. 14, 2002, pages 503 - 508, XP008037861
- SEIDEL G.E.: 'Economics of selecting for sex: the most important genetic trait' THERIOGENOLOGY vol. 59, 2003, pages 585 - 598, XP002981959

## Description

### CROSS-REFERENCES TO RELATED APPLICATIONS

This application claims the benefit of U.S. provisional application number 60/410,884, filed 13 September 2003 and U.S. non-provisional application number 10/340,881, filed 09 January 2003.

### TECHNICAL FIELD

The present invention relates to sperm cell preservation, and the processing of sperm cells, generally. The invention may be applicable to artificial insemination, in vitro fertilization, super-ovulation, mammal production, sperm samples, and sperm cell processing, including processing such as collecting, handling, sorting, storage, transportation, use, fertilization, or insemination features.

### BACKGROUND OF THE INVENTION

The fertilization of mammal eggs, potentially referred to as oocytes, such as by artificial insemination and in vitro fertilization techniques have been conducted in attempt to increase the fertility rates of livestock. Furthermore, effective pre-selection of sex has been accomplished in many species of livestock following the development of safe and reliable methods of sorting, generally, or specifically separating sperm cells into enriched X chromosome bearing and Y chromosome bearing populations. Separation of X chromosome bearing sperm cells from Y chromosome bearing sperm cells, as well as collection, handling, sorting, storage, transportation, use, fertilization, or insemination techniques, or sperm cell and semen processing generally, can be accomplished as disclosed herein and as disclosed by various patent applications, for example: PCT/US99/17165; PCT/US01/45023; PCT/US01/15150; PCT/US98/27909; PCT/US01/45237, PCT/US01/18879, PCT/US00/30155, PCT/US01/02304, U.S. Patent Number 6,071,689, Patent Number 6,372,422, U.S. divisional application number 10/081,955, U.S. provisional application number 60/400,486, and U.S. provisional application number 60/400,971.

Although the various devices and methods of sperm cell processing, generally, and the collection, handling, sorting, storage, transportation, usage, fertilization, and insemination of sperm cells have been improved over the past several years, significant problems remain with respect to maintaining sperm quality, such as viability, motility, and functionality, and preservation and stimulation of sperm cells, especially with regard to artificial insemination (in vivo) and in vitro fertilization (IVF) procedures. One potential consequence is the reduction in fertilization rates. Sperm quality, such as the viability of sperm separated into enriched X-chromosome bearing and Y-chromosome bearing populations could be compared directly in-vitro (for example, in conjunction with IVF procedures) and in-vivo (for example, in conjunction with artificial insemination procedures), is generally reduced during traditional sperm cell processing. More generally, traditional processing techniques addressing sperm viability, motility, functionality, preservation, stimulation, fertilization, and insemination may have not yielded preferred or even adequate fertilization rates or sperm quality, for example.

As one example of traditional sperm processing, techniques of sperm cell sorting for the breeding of mammals may be limited, for example, with regard to sperm cell quality, such as sperm cell viability, motility, and functionality, and fertilization rates as well as potentially other limited characteristics or features. For example, if the sorter apparatus is a relatively long distance from the males, or if the sorter apparatus is a relatively long distance from the receiving females to be inseminated or eggs to be fertilized with processed sperm, the sperm cell quality, fertilization rates, or other characteristics or features may be adversely affected. Some of the techniques or some traditional techniques may achieve to some degree sperm cell viability, motility, or functionality, and desirable fertilization rates, while addressing, for example, sperm preservation during transportation to the sorter apparatus. However, further techniques achieving sufficient sperm cell quality, such as viability, motility, and functionality, and other features such as sperm cell stimulation, preservation, and maintained or enhanced fertilization rates, may be desirable, especially for any one or a combination of various sperm processing steps. The processing features may include, for example, collection, handling, sorting, storage, transportation, usage, fertilization, or insemination, and especially the preservation of the sorted sperm cells from the sorter apparatus to the site of fertilization (potentially via collected individual samples or "straws"), or at any other stage of sperm processing.

As another example of the limitations of traditional sperm processing, traditional processing techniques may be limited with regard to sperm cell quality, generally, such as sperm cell viability, motility, and functionality, and sperm cell stimulation, sperm cell preservation, and fertilization rates, due to the type and the extent of processing involved. Known processing techniques such as preservation or sorting, for example, may degrade sperm quality, and further reduce fertilization rates. The degradation of sperm quality and fertilization rates may have previously proven problematic in circumstances that may have required further steps of sperm cell preservation, such as in circumstances wherein the sorter apparatus is a relatively long distance from the males or when the sorter apparatus is a relatively long distance from the receiving females to be inseminated or otherwise fertilized with processed sperm. However, the potentially identified degradation of sperm quality and fertilization rates may not have been heretofore addressed in the context of sperm cell preservation, or even identified as related to sperm cell preservation, or sperm cell processing generally, as traditional preservation techniques may have actually been understood to have contributed to the adverse effects to sperm quality, fertilization rates, or other relevant characteristics or results.

It may have been traditionally understood that additional processing steps in the processing of sperm cells or semen having a concentration of sperm cells would degrade sperm quality and reduce fertilization rates to such an extent that additional processing steps, generally, would be avoided. Specifically, it may also have been traditionally understood that processing steps such as preservation, specifically cryopreservation, and sorting might be too damaging to the sperm cells, especially in processing sequences incorporating both cryopreservation and sorting. Furthermore, it may have even been suggested and taught in traditional methods that preservation of sperm, such as cryopreservation, provided after previous processing steps so as to preserve the sperm for later procedures, such as for in vivo or in vitro techniques, could not be accomplished without compromising the sperm cells and the technique itself to such a degree that the results of the later procedures would be detrimentally affected. Accordingly, such concerns may have even been demonstrated in traditional sperm cell processing procedures, teaching away from subsequent processing steps such as sorting and cryopreservation, or processing steps incorporating multiple cryopreservation steps.

### DISCLOSURE OF THE INVENTION

The present invention may address the variety of previously identified and potentially unaddressed problems associated with reduced sperm cell quality, such as viability, motility, and functionality, and stimulation, preservation, and other sperm cell characteristics or features. Sperm cells that may have been or will be processed by one or more steps of collection, handling, sorting, storage, transportation, usage, fertilization, or insemination and potential reductions fertilization rates are further addressed. The instant invention may also address the variety of problems associated with sperm cell quality of sperm cells that have been separated into enriched X-chromosome bearing and Y-chromosome bearing populations, potentially sperm cells sorted through techniques such as flow sorting, and the potential reduction in fertilization rates. More generally, the present invention may address traditionally low fertilization rates of in vivo artificial insemination and in vitro fertilization, and sperm cell quality issues such as sperm cell viability, motility, and functionality. The present invention also addresses issues such as stimulation, preservation, and fertilization rates, as well as enhanced or maintained fertility characteristics, such as blastocyst development rate, in vivo developmental capacity, pregnancy rate, and cell division, achieving results that may have been unexpected to those skilled in the art. Additionally, the present invention addresses numbers of processed sperm potentially needed to obtain desired pregnancy rates, and further in consideration of pregnancy rates obtained respective of sorted and unsorted preserved sperm.

Naturally, further significant objects and goals of the invention are disclosed in the proceeding description of the invention.

Accordingly, to achieve the various objects and goals of the invention, the present invention provides a method of processing a mammal sperm sample according to claim 1, the steps comprising obtaining sperm cells, cryopreserving the sperm cells, thawing the sperm cells, processing the sperm cells, and cryopreserving the sperm cells. Corresponding method of mammal production and mammals and sperm samples are also disclosed.

Furthermore, embodiments of the invention may comprise a method of sperm cell processing, and the steps provided as obtaining sperm cells, cryopreserving the sperm cells, thawing the sperm cells, processing the sperm cells; and cryopreserving the sperm cells. Further, a method of producing a mammal, comprising the method steps of obtaining sperm cells, cryopreserving the sperm cells, thawing the sperm cells, sorting the sperm cells, cryopreserving the sperm cells, thawing the sperm cells, fertilizing at least one egg, and producing a mammal from the at least one fertilized egg, is disclosed but not covered by the scope of the claims.

Furthermore, to achieve the various objects and goals of the invention, the present invention may be directed to embodiments providing for artificial insemination (in vivo) and in vitro fertilization procedures, and in some embodiments directed to super ovulation procedures. Additionally, embodiments may be further directed to establishing sperm samples such as straws, pellets, insemination doses, or other prepared sperm samples, and the processing of semen.

Additional embodiments of the invention are disclosed throughout the description of the invention and in the following claims.

### BRIEF DESCRIPTION OF THE DRAWINGS

Figure 1 is a flow diagram of one embodiment of the present invention.
Figure 2 is a flow diagram of a second embodiment of the present invention.
Figure 3 is a flow diagram not covered by the scope of the claims.
Figure 4 is a flow diagram of an embodiment of the present invention.
Figure 5 is a flow diagram not covered by the scope of the claims, potentially provided in combination with the embodiment of Figure 1.

### MODES FOR CARRYING OUT THE INVENTION

The present invention discloses semen and sperm cell processing and preservation techniques and preservation, stimulation, fertilization, and insemination addressing one or more sperm cell characteristics or sperm quality, such as sperm cell viability, motility, and functionality, and stimulation, preservation, and fertilization rates. Embodiments of the present invention further disclose enhanced or maintained fertility characteristics, such as blastocyst development rate, in vivo developmental capacity, pregnancy rate, or cell division. Sperm cell characteristics or sperm quality may be addressed within the context of various processing techniques, such as collection, handling, separation, storage, transportation, usage, fertilization, or insemination.

Sperm cell quality may be any one or a combination of the various attributes of sperm cells previously mentioned or further mentioned herein, such as, for example, viability, motility, and functionality and may even be considered one or a combination of fertility characteristics of the sperm cells. Sperm cell characteristic may refer to any one or a combination of various biological, chemical, physical, physiological, or functional attributes of one or more sperm cells, such as chromosome bearing attributes of the cell, or in some embodiments may refer to sperm cell quality as previously described.

Fertility characteristics may be enhanced or maintained, and may comprise blastocyst development rate, in vivo developmental capacity, pregnancy rate, or cell division.

Semen or sperm cell processing techniques may refer to any one or a combination of preservation, stimulation, insemination, fertilization, sorting, selection, separation, or thawing, and may be specifically directed to any one or a combination of collection, handling, selection, storage, transportation, usage, fertilization, or insemination techniques.

Sperm samples may refer to a volume containing sperm cells, potentially comprising semen, carrier fluid or other materials, and may comprise one or a plurality of pellets, straws, insemination doses or other prepared sperm samples.

Sperm cells may be maintained or enhanced in accordance with the present invention, and in some embodiments provided as sperm cell preservation or, more broadly, sperm cell processing, such as through processing steps as in sperm cell sorting or sperm cell preservation, and may be embodied in accordance with fertilization and insemination techniques. Embodiments may comprise some component features of preservation of sperm cells or other processing techniques as may be disclosed in the previously mentioned patents and patent applications, and as further described below.

Sperm cells may also be maintained or enhanced in some embodiments by preservation and stimulation techniques as further described below, and also in further combination with the processing, stimulation, preservation, fertilization, and insemination techniques in the previously mentioned patent applications and patents.

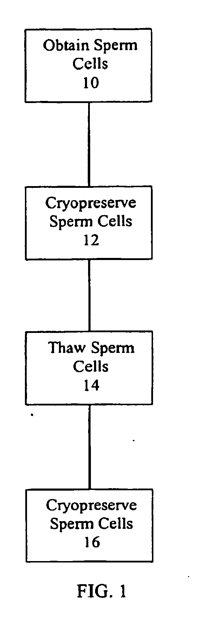

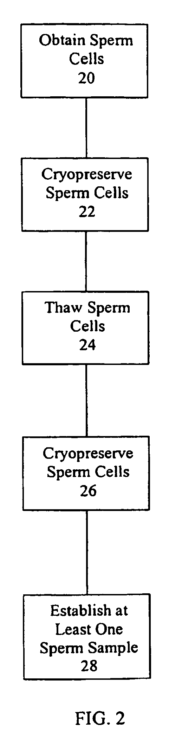

Accordingly, the present invention is embodied as methods of sperm cell processing according to claim 1. Referring to figure 1, the steps of sperm cell processing of some embodiments are shown in flow diagram. Sperm cells are obtained 10 and the sperm cells are cryopreserved 20. The sperm cells are then thawed 14 and a subsequent step of cryopreservation 16 is performed. Each of these features is further described below. A sperm cell processing embodiment of the invention consistent with the features shown in Figure 1 is shown in Figure 2. The embodiment provides steps 20, 22, 24, and 26 consistent with the embodiment of Figure 1, and further establishes at least one sperm sample. The sperm sample may comprise any sperm sample as previously described and consistent with the further description that follows.

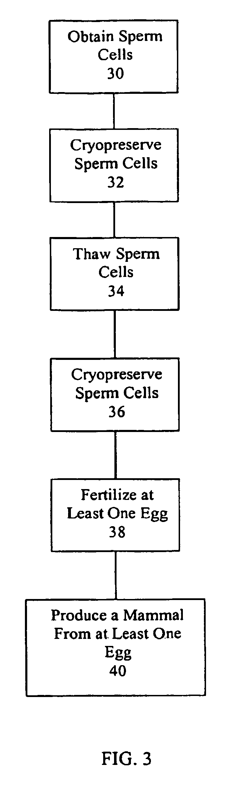

Figure 3 shows methods of producing a mammal not covered by the scope of the claims consistent with the embodiments of Figure 1, as may be most easily identified in steps 30, 32, 34, and 36 of Figure 3. The sperm cells from feature 36 fertilize at least one egg 28 and a mammal is produced 40 from the at least one egg. Again, each of these features is further described below.

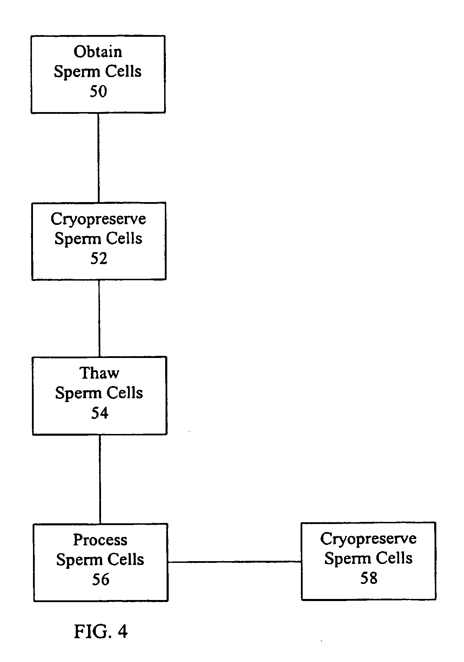

Figure 4 shows embodiments of sperm cell processing in accordance with the present invention. As shown, and in further elaboration on the previously described features, the sperm cells are obtained 50 from a mammalian source, either directly or in various combinations of steps, such as through a storage facility. The sperm cells may be cryopreserved 52. Cryopreservation may be provided as further described in some embodiments of the invention below. Subsequently, the sperm cells may be thawed 54. Further processing 56 may then be performed on the sperm cells prior to another cryopreservation of the sperm cells 58.

Sperm cell processing, generally, and the processes previously described may be performed independent of insemination and fertilization techniques, or as a part of and in combination with such techniques. Each of the above processes may also be performed respective of super ovulation techniques.

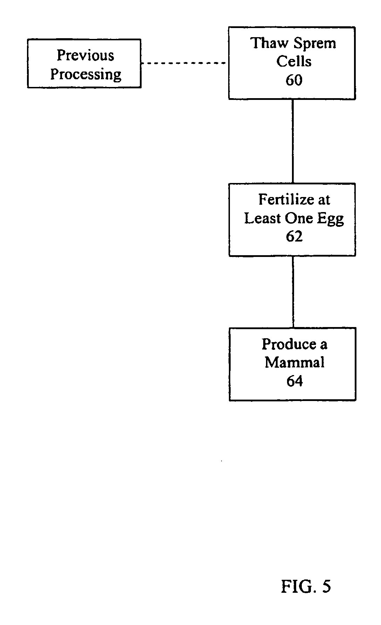

Methods of producing a mammal are shown in Figure 5 not covered by the scope of the claims. After previous processing, or as proceeding with the features 50 through 58, at least one egg may be fertilized 62. The sperm cells may be thawed 60 and the fertilization 62 of at least one egg with the sperm cells may be conducted. A mammal may then be produced 64 from the egg fertilized by the processed sperm cells. Additional processing steps such as sorting of the sperm cells is provided and is preferably performed after the first thaw (54).

Furthermore, embodiments of the invention such as those previously described support in vivo and in vitro techniques. Accordingly, a female of the species of the mammal from which sperm was obtained may be inseminated with the sperm cells, such as the previously processed sperm cells, or sorted and cryopreserved sperm cells, each previously described. Insemination techniques may comprise inseminating the at least one female with the sperm cells, the provision of which may be either thawed or potentially as cryopreserved sperm cells in an unthawed condition, potentially in pellet or other sperm sample configuration as previously described. Thawed sperm samples may be preferred if, for example, sperm cell quality is to be maintained or enhanced as part of the sperm cell processing or mammal production. However, unthawed cryopreserved cells could be used for insemination and preferred, if, for example, a thaw of the sperm cells within the female is acceptable, and if a reduction of steps is desired outside the womb, such as to reduce sperm cells processing. Furthermore, artificial insemination and in vitro fertilization techniques, generally, may be accomplished with unthawed cryopreserved cells, independent of or in combination with other processing features such as thawing, subsequent steps of cryopreserving, sorting, or other features presently disclosed. Processing steps such as sorting of the sperm cells may also be performed.

Insemination may be performed with at least one sperm sample such as those previously described. At least one egg of the female may be fertilized and a mammal produced from the at least one fertilized egg. In accordance with in vitro procedures, fertilizing in vitro at least one egg may be performed. The at least one fertilized egg may be transferred to a recipient female, and a mammalian embryo may be produced, or, a mammal may be produced, from the at least one fertilized egg.

Super ovulation techniques are also disclosed. A female of the species of mammal may be superovulated. The superovulated female may be inseminated with the sperm cells and at least one egg fertilized. At least one egg of the superovulated female may be obtained and the at least one egg fertilized in vitro. The at least one fertilized egg may be transferred to a recipient female in either of the artificial insemination or in vitro fertilization techniques.

As previously mentioned, methods of sperm cell processing may comprise the establishment of at least one sperm sample of the sperm cells. Accordingly, embodiments of the invention may comprise methods of sperm cell processing consistent with embodiments previously described, such as those shown in Figure 2, that feature establishing at least one sperm sample of the sperm cells. In some embodiments, the at least one established sperm sample may be the subject of a subsequent cryopreservation step, such that the at least one sperm sample is cryopreserved.

It may have been previously thought that consistent with conventional processing techniques that such processed sperm cells, and established sperm samples, would not be of sufficient sperm quality or otherwise not be capable of fertilization. Some preferred embodiments of the present invention, however, also provide that the sperm sample be capable of fertilization of at least one egg of a female mammal, as may be shown in the various embodiments described below, and further the insemination of such female with the at least one sperm sample, and in some embodiments, the fertilizing of at least one egg of a female mammal with the at least one sperm sample. The feature of sperm samples capable of fertilization extends in some embodiments to capability of fertilization of a species of a superovulated female mammal, and the insemination of such superolvulated female with the at least one sperm sample. The feature of sperm samples capable of fertilization also extends in some embodiments to capability of fertilization in vitro of at least one egg, and the fertilization in vitro of at least one egg with the at least one sperm sample. Further embodiments provide for establishing at least one sperm sample capable of inseminating at least one female mammal, and the insemination of such at least one female with said at least one sperm sample. The fertilization of a plurality of eggs, and even the production of a mammalian embryo, an embryo that may be

transferred to a recipient female, and more generally the production of a mammal, such as mammalian offspring, are provided consistent with the fertilization capability of the sperm sample and the additional features described above. Wherein addressing sperm sample capability further disclosed is producing at least one animal of a pre-selected sex from said cryopreserved processed sperm cells. Again, sperm samples may accordingly be established and may be configured as a straw, a pellet, an insemination dose, or a prepared sperm sample.

Each of the embodiments previously described, and those further described below, may be considered departures from traditional sperm cell processing, the production of mammals, the production of mammalian embryo, and the establishment of sperm samples. It has been traditionally viewed that such processing of sperm cells could not sufficiently ensure sperm quality or provide adequate rates of insemination, fertilization, or pregnancy. However, and as been taught in the various references cited herein , sperm cells may in fact be processed to achieve, for example, the sorting of sperm cells, potentially to differentiate sperm cells as either X chromosome bearing or Y chromosome bearing sperm cells, in some instances to provide for preselection of the sex of a mammal or mammalian embryo. The present invention also provides for such processing, and in some preferred embodiments, additional features of preservation heretofore traditionally thought not to be feasible commercially, or even possible, and heretofore providing a solution to those previously identified but unaddressed issues

Semen, and in particular sperm cells, are obtained and otherwise heretofore processed from mammals in accordance with the present invention such as equids, bovids, felids, ovids, canids, buffalo, oxen, elk, or porcine, or other mammal species. Further, some embodiments may provide obtaining and processing semen or sperm cells from prized mammal species, endangered mammal species, rare individuals of a mammal species, and even from zoological specimens or individuals. The resulting mammal or mammalian embryo may be produced in accordance with the techniques as previously described and as further described below. Sperm samples may be established, in some embodiments, as previously described.

Sperm samples are cryopreserved, such as by freezing, using various preservation techniques, such as freezing in a Hepes-buffered crydiluent. Sperm cells may be provided as pellets or straws and may be thawed using various thawing techniques, for example, with ram spermatozoa. Other sperm samples may be provided throughout the processing of sperm cells, and may or may not be cryopreserved when established as a sperm sample or when provided to inseminate or fertilize an egg.

One or more additives, singularly or in combination, may be introduced into the semen, sperm cells, or sperm sample. In one embodiment, a cryodiluent may be introduced into the sperm sample to preserve the sperm cells. The introduction of the additive or additives, such as a cryodiluent, into the sperm sample, and in some embodiments with as a cryopreservation step, potentially referred to as freezing, may maintain or enhance sperm cells, and may further maintain or enhance sperm quality, sperm cell quality, such as sperm cell viability, motility, and functionality, and may maintain or enhance stimulation and fertilization rates, and potentially one or more sperm cell characteristics. In other embodiments of the present invention, an additive or additives, such as cryodiluent, singularly or in combination, could be removed from the sperm sample. The removal of the cryodiluent or other additives from the sperm sample, and in some embodiments with cryopreservation steps, may maintain or enhance sperm cells, and may further maintain or enhance sperm quality, such as maintaining or enhancing sperm cell viability, motility, and functionality, and fertilization rates, and potentially one or more sperm cell characteristics.

Sperm cells may also be maintained or enhanced in some embodiments of the present invention as part of the disclosed techniques, and sperm quality may further be maintained or enhanced, such as maintaining or enhancing sperm cell viability, motility, and functionality, and potentially one or more sperm cell characteristics. Furthermore, fertilization rates may be at least maintained, and even enhanced, in accordance with the features of the present invention and as further described below. Fertilization characteristics may also maintained or enhanced, and in some embodiments blastocyst development rates may be enhanced, monospermic fertilization at least maintained and even enhanced, and cleavage rates at least maintained and even enhanced. In some embodiments of the invention, the sperm sample may be preserved, as previously described, such as through cryopreservation, such as freezing, or other preservation techniques such as those disclosed in the previously mentioned patent applications and patents . Sperm quality is maintained in the embodiments as further described below. Maintenance of sperm quality, however, should be construed as encompassing enhancement features associated with the sperm cells. For example, the present invention may provide enhanced motility, enhanced viability, and enhanced functionality of the sperm cells, and in some embodiments the sperm cells of a sperm sample.

Accordingly, cryodiluent or other additives, singularly or in combination, may be introduced into the sperm sample to preserve or stimulate the sperm. In one preferred embodiment, the introduction of the cryodiluent or other additives into the sperm sample contributes to the preservation of the sperm through cryopreservation, freezing the sperm sample, and therefore maintaining or enhancing the sperm cells, and further maintaining or enhancing sperm quality, such as at least maintaining or even enhancing sperm cell viability, motility, and functionality, and potentially one or more sperm cell characteristics. As previously mentioned, the cyrodilutent or other additives may be introduced into the sperm sample prior to sample preservation or as sample preservation. The sample may then be processed such as through collection, handling, sorting, storage, transportation, usage, fertilization, or insemination techniques as disclosed in the previously mentioned patent applications and patents.

In one embodiment, cryodiluent or other additives, singularly or in combination, may be introduced into a sperm sample previously collected and the sample cryopreserved, such as through freezing, and followed by a thaw of the sample and subsequent processing, including collection, handling, sorting, storage, transportation, usage, fertilization, or insemination processing techniques. One such processing step may be provided as sorting, and in some embodiments as the separation of X chromosome bearing sperm cells from Y chromosome bearing sperm cells, potentially into a high purity population sample or samples. Various benefits may be achieved through such a sperm processing technique, including the maintenance of sperm quality. For example, various methods of sorting as described in the previously mentioned patent applications and patents achieve a separation of X chromosome bearing sperm cells from Y chromosome bearing sperm cells while minimizing damage to the viable sperm cells. Further, non-viable sperm, contamination, or crydiluent or other additives, for example, may be eliminated through such separation techniques. Additionally, other aspects of sperm quality may be maintained or enhanced, particularly that of sperm cell viability, motility and functionality. The sperm sample may further be stimulated during the processing to maintain or enhance sperm quality as previously described. One such method of sperm processing known in the art is described in U.S. Patent No. 5,135,759, , disclosing a flow cytometer sorting technique.

The processing of sperm cells may be performed by sorting the sperm cells as previously described, or in some embodiments, in accordance with the following process. Sorting may comprise, in some embodiments, as a selecting of sperm cells based upon at least one desired characteristic, potentially sperm cell quality characteristics, such as viability, motility, functionality, stimulation, and preservation, or one or a combination of various sperm cell characteristics such as biological, chemical, physical, physiological, or functional attributes of one or more sperm cells, such as chromosome bearing attributes of the cell. The sperm cells may be stained, preferably with Hoechst 33342 or like stain or dye, or in combination with such stains or dyes, and the sperms cells differentiated based upon the staining and selection. The cells may then be separated based upon the differentiation and then collected. The sperm cells may be so processed in accordance with the various techniques of those references cited herein and in some preferred embodiments, processed to so comprise the maintenance of sperm quality.

### EXAMPLE EMBODIMENT 1

In one embodiment of a preserving and processing technique, 200µl of thawed spermatozoa was placed onto a 2ml separation gradient (90%:45%) of Puresperm™, a human preparation, and a Tris based diluent. The gradient preparations were then centrifuged at 1000g for 15 minutes. The post-Puresperm™ pellet was removed, slowly diluted 1:4 with warm Tris based diluent and centrifuged at 650g for 3 minutes. The supernatant was removed and the sperm stained, incubated and sorted as previously described. The Tris based diluent was used as the staining medium and Androhep® (Minitub, Germany) plus 20% egg yolk was used as the collection medium.

The above example is one of various embodiments of the inventive technique, providing preservation of sperm, such as through cryopreservation or freezing, thawing the sperm, identifying the sperm, such as through staining, and sorting the sperm, such as into X and Y chromosome bearing populations.

Although the previous example provides a sperm preservation technique and processing technique of sperm collection, preservation, thaw and separation, other techniques are encompassed by and explicitly disclosed in the present invention. For example, one or a combination of various collection, handling, separation, storage, transportation, usage, fertilization, or insemination techniques may be performed as part of this present inventive technique. In one embodiment, sperm may be collected, followed by separation of X chromosome bearing sperm cells from Y chromosome bearing sperm cells. A preservation step prior to sperm cell separation may not be needed, for example, during circumstances in which the sorter apparatus is readily available after sperm collection. The sorted sperm sample or samples may then be preserved as an additional step and as previously described, potentially for further handling, separation, storage, transportation, usage, fertilization or insemination. The sorted sperm sample may further be stimulated during the processing to maintain or enhance sperm quality as previously described. The next example describes one example of a processing technique.

### EXAMPLE EMBODIMENT 2

Sorted samples were centrifuged at 700g for 6 min. at room temperature (24C). The supernatant was removed and the sorted sperm could be used "fresh" for AI or in an IVF system; or the remaining pellet was extended 1:4 with the Hepes based cryodiluent, potentially the same diluent used for an original cryopreservation of the sperm, and frozen using various techniques, such as those previously described and as described below. The re-frozen and sorted sperm were thawed using methods such as a glass tube shaken in a 37°C waterbath, and then could be used in an AI or IVF system.

### EXAMPLE EMBODIMENT 3

The use of spermatozoa sorted from frozen-thawed pellets in the ovine mammal regarding in vitro fertilization systems. Frozen-sorted and frozen-sorted-frozen sperm used in an ovine IVF system were slowly diluted with 0.5ml of IVF media, and in some embodiments, using ovine IVF protocol, and centrifuged in a Falcon tube with a tight lid at 300g for 6 minutes. The supernatant was removed and the remaining sperm quickly placed in the IVF well at a concentration of one million motile sperm/ml. Preferably, a high standard of media preparation (i.e. use of eggs less than 24 hours old, ultracentrifugation of egg yolk diluents and meticulous filtering) and handling of the samples (i.e. constant temperature) is required.

Other preservation, processing, fertilization, and insemination techniques need not include a separation step. For example, the sperm may be collected, followed by a preservation step and subsequent handling, storage, transportation, usage, fertilization, or insemination.

Furthermore, one or more preservation steps may be conducted as part of preservation, processing, fertilization, and insemination techniques, or combinations thereof, such preservation in some embodiments comprising cryopreservation, such as through freezing of the sperm sample, and subsequent steps of thawing, occurring before, concurrent with, or after one or more other processing techniques.

### EXAMPLE EMBODIMENT 4

Application of sperm sorting to breeding of livestock and wildlife may be limited when the sorter is a long distance from the male(s), as previously described, but would be facilitated by the sorting of cryopreserved and thawed sperm, such as frozen-thawed sperm (Lu KH et al., Theriogenology 1999:52:1393-1405) and cryopreserving or refreezing it. High purity sorting with maintained quality of frozen-thawed ram sperm may be achieved after processing to remove the cryodiluent. The aim of this study was to evaluate the functional capacity of frozen-thawed sperm after sorting and a second cryopreservation/thawing step. Frozen semen from 2 rams (n=2 ejaculates per ram) was used throughout Post-thaw sperm treatments comprised (i) unsorted (Control); (ii) sorted (Frozen-Sort) and (iii) sorted then re-frozen (Frozen-Sort-Frozen). X and Y sperm were separated using a high-speed sorter (SX MoFlo(R), Cytomation, CO, USA) after incubation with Hoechst 33342 and food dye to eliminate non-viable sperm. Reanalysis revealed high levels of purity for X- and Y-enriched samples for all treatments (87.0 +/-4.5%). For IVF, 472 IVM oocytes were inseminated with 1 x 10(6) motile sperm/mL. After 3 h in SOF medium, oocytes were transferred to Sydney IVF cleavage medium (Cook(R), QLD, Australia) for 4 d followed by Sydney IVF blastocyst medium (Cook(R)) for an additional 3 d culture in 5% O2: 5%CO2: 90% N2. Oocytes were assessed for cleavage at 24 and 48 h post-insemination (p.i.). At 52 h p.i., uncleaved oocytes were stained with orcein for assessment of maturation and fertilization. Data from 3 replicates were analyzed by ANOVA, Chi-square and Fisher Exact Test. At insemination, % motile sperm (+/- SEM) was higher (P<0.001) for Frozen-Sort (85.8 +/- 2.4%) and Frozen-Sort-Frozen (66.7 +/- 7.7%) than Control (36.7 +/- 2.1%). Maturation rate was 95.6% (451/472). Cleavage of oocytes in a parthenogenetic control group (no sperm) was low (2/56; 3.6%). Polysperminc fertilization was low (9/451; 2.0%) and did not differ among treatments.

**Table 1. Fertilization & early embryo development of oocytes after incubation with frozen-thawed unsorted (Control), frozen-thawed & sorted (Froz-Sort) & frozen-thawed, sorted then frozen-thawed (Froz-Sort-Froz) ram sperm. Values in parentheses are percentages.**

| Treatment | No. of mature oocytes fertilized^{d} | No. of mature oocytes undergoing cleavage after insemination | | No. of cleaved oocytes forming blastorysts | | |
|---|---|---|---|---|---|---|
| | | 24h | 48h | Day 5 | Day 6 | Day 7 |
| Control | 40(67.8) | 26(44.1) | 36(61.0) | 4(11.1) | 13(36.1) | 16(44.4)' |
| Froz-Sort | 110(63.6) | 67(38.7) | 109(63.0) | 24(22.1) | 34(31.2) | 57(52.3))" |
| Froz-Sort-Froz | 94(57.7) | 71 (43.6) | 91(55.8) | 23(25.3) (64.8)^{bc} | 347(40.7) | 59 |

| | | | | | | |
|---|---|---|---|---|---|---|
| ^{d}Monospermic fertilization. within column, values with different superscripts differ (P<0.05). | | | | | | |

Fertilization and cleavage rates were consistently high across treatments. Blastocyst development rate was higher for oocytes fertilized with Froz-Sort-Froz than with Control sperm. These results demonstrate that frozen-thawed ram sperm can be sex-sorted for either immediate or future use in an IVF system after re-cryopreservation.

The above example is one of various embodiments of the inventive technique, providing preservation of sperm, such as through cryopreservation, such as freezing, thawing the sperm, sorting the sperm, such as into X and Y chromosome bearing populations, preserving the sorted sample, such as through cryopreservation or freezing, and further including thawing the sperm sample for use, such as for fertilization or insemination.

Consideration should be given to fertility rates respective of preservation and processing procedures, such as separation for sex-sorting and cryopreservation of sperm samples, as the next Example describes, and in combination with cryopreservation, thawing, processing, and subsequent cryopreservation, as disclosed in this description.

### EXAMPLE EMBODIMENT 5

Lambs have been produced after artificial insemination (AI) with low numbers (2-4x10⁶) of cryopreserved sex-sorted sperm. Fewer ewes were pregnant after AI with X- or Y-sorted frozen-thawed (25%, 15% respectively) than with a commercial dose of unsorted frozen-thawed sperm (54%). The object of the present study was to determine the minimum numbers of sorted frozen-thawed sperm required to obtain pregnancy rates similar to those obtained with unsorted sperm.

A sample of sperm from single ejaculates of 2 rams was stained, incubated, analyzed and sorted using a modified high speed cell sorter (MoFlo®, Cytomation, Fort Collins, CO, USA) as previously described. Sperm were processed at 15,000-18,000/serc without sex-sorting into 10ml centrifuge tubes pre-soaked with 1% BSA in sheath fluid containing 0.2ml Tris-buffered medium and 20% egg yolk (v/v). For every sample, 1.3 x 10⁶ sperm were sex-sorted and analyzed to determine purity. Sorted and unsorted (control) samples were extended with a zwitterions-buffered diluent containing 13.5% egg yolk and 6% glycerol and frozen as 250ul pellets containing 5x10⁶ sperm. The time of estrus was controlled in 144 Merino ewes by progestagen sponges (FGA, Vetrepharm A/Asia, Sydney) inserted intravaginally for 12 days and an injection of 400 I.U of PMSG (Pregnecol, Vetrepharm A/Asia) at sponge removal (SR). Thirty-six h after SR 134 ewes were injected with 40µg GnRH (Fertagyl®, Intervet) to control the time of ovulation. One hundred and eleven ewes were inseminated in the uterus by laparoscopy 57-60h after SR with 5, 10, 20 or 40x10⁶ sorted or unsorted frozen-thawed sperm. Thirteen ewes not given GnRH were inseminated with a commercial dose of unsorted frozen-thawed sperm. Thirteen ewes not given GnRH were inseminated with a commercial dose of unsorted frozen-thawed sperm 57-58h after SR. Pregnancy was diagnosed by ultrasound on d53. The data were analyzed by Chi-square.

Sperm motility after thawing was 37.8+/-1.78% (sorted) and 42.9+/-0.93% (unsorted). Seven of 13 (53.8%) ewes not given GnRH were pregnant. Of the GnRH-treated ewes the proportion pregnant was affected by the number of sperm inseminated (p<0.05) but not by ram or type of sperm (p>.0.05). For ewes inseminated with sorted or unsorted (control) frozen-thawed sperm, pregnancy rate was higher for inseminates of 10 and 40x10⁶ than for 5 and 20x10⁶ sperm (Table 1). The results suggest that a minimum of 40x10⁶ sorted frozen-thawed sperm inseminated close to the time of ovulation are required to obtain commercially acceptable pregnancy rates.

**Table 1: Pregnancy after intrauterine insemination of ewes with frozen-thawed control and sorted ram sperm.**

| Dose (x 10⁶ sperm) | No. ewes inseminated | No. ewes pregnant | % ewes pregnant |
|---|---|---|---|
| 5 | 30 | 10 | 33.3^{a} |
| 10 | 28 | 16 | 57.1^{b} |
| 20 | 29 | 10 | 34.5^{a} |
| 40 | 23 | 16 | 69.6^{a} |

| | | | |
|---|---|---|---|
| ^{ab}Within columns different superscripts differ (p<0.05). ^{a}This research was supported by XY, Inc; CO, USA; The Australian Research Council, Vetrephann A/Asia. | | | |

### EXAMPLE EMBODIMENT 6

The ability to sort and re-freeze frozen-thawed sperm would significantly increase the potential application of sperm processing technology such as sperm sexing technology to applications such as species management. Frozen-thawed, sorted, re-frozen then thawed ram sperm, in accordance with some embodiments of the invention, appear fully functional in vitro with blastocyst production greater than that for frozen-thawed, non-sorted sperm. (Hollingshead FK et al. 2003 Theriogenology 59 209). Some embodiments may be especially useful to evaluate the in vitro capacity of in vitro produced embryos derived from frozen-thawed sperm after sorting and a second cryopreservation/thawing step.

Frozen semen from 2 rams (n=3 ejaculates per ram) was used throughout one technique of the present invention. Post-thaw sperm treatments comprised (i) non-sorted (Control); (ii) sorted (Froz-Sort) and (iii) sorted then re-frozen (Froz-Sort-Froz). X and Y sperm were separated using a high-speed sorter (SX MoFlo®, DakoCytomation, Fort Collins, CO, USA) after incubation with Hoechst 33342 and food dye to eliminate non-viable sperm. Reanalysis in some embodiments revealed high levels (mean ± SEM) of purity for X- and Y-enriched samples for all treatments (89 ± 1.2%). At Day 6 post-insemination in one performed technique of the invention, 2 embryos (blastocyst stage or greater) were transferred per recipient. Data were analyzed by Chi-square and Fisher Exact Test. In vivo embryo survival for this embodiment may be similar across sperm treatments (28/64, 43.8% overall) and 20 of 23 (87.0%) sexed lambs were of the predicted sex (Table 2). These results of embodiments of the invention may demonstrate high in vivo developmental capacity of in vitro produced sexed embryos derived from frozen-thawed ram sperm after sorting and a second cryopreservation/thawing step, and increase the potential application of sperm processing technology such as sexing technology.

**Table 2: In vivo survival of transferred in vitro produced embryos derived from frozen-thawed non-sorted (Control), frozen-thawed & sorted (Froz-Sort) & frozen-thawed, sorted then frozen-thawed (Froz-Sort-Froz) ram sperm.**

| Sperm treatment | No. recipients | No. pregnant on Day 20 (%)^{a} | No. pregnant on Day 60 (%)^{b} | No. pregnancies lost between Day 20 and Day 60 (%) | No. lambs born/ no. of transferred embryos (%) |
|---|---|---|---|---|---|
| Control | 8 | 6 (75.0) | 5 (62.5) | 1 (16.7) | 5/16 (31.3) |
| Froz-Sort | 9 | 7 (77.8) | 5 (55.6) | 2 (28.6) | 6/18 (33.3) |
| Froz-Sort-Froz | 17 | 14 (82.4) | 14 (82.4) | 0 (0) | 17/34 (50.0) |

| | | | | | |
|---|---|---|---|---|---|
| ^{a}Diagnostic by blood progesterone assay. ^{b}Diagnosed by ultrasonography. | | | | | |

Further disclosed are sperm samples as previously defined, such as a straw, and in some embodiments straws utilized in IVF, produced in accordance with any of the above described embodiments of the invention, such as any of the processing, production of mammal, production of mammal embryo, stimulation, preservation, fertilization, and insemination techniques, and be of maintained or enhanced sperm quality, such as a straw of desired viability, motility, and functionality, or other characteristics, or combinations of characteristics, such as characteristics disclosed herein, potentially resulting in desirable levels of fertilization rates, and in some embodiments, particularly for equine mammals. The sperm sample such as at least one or a plurality of straws may be particularly suited for individual production of embryos.

The invention further includes various processing, preservation, stimulation, fertilization, insemination, techniques as disclosed herein and as may comprise in combination features disclosed in the previously mentioned patent applications and references. Accordingly, the various semen and sperm cell processing features provided as systems of preservation, stimulation, fertilization, insemination, embodiments, address sperm quality such as one or more sperm cell characteristics, such as viability, motility, functionality, or fertilization rates consistent with the disclosures of the previously mentioned patent applications and references. Further, sperm cell characteristics may be addressed within the context of various collection, handling, separation, storage, transportation, usage, fertilization, or insemination techniques, and in those or other various embodiments, within the context of assaying, testing, or determining the biological, chemical, physical, physiological, or functional attributes of sperm cells. Therefore, systems of the present invention may provide sperm cell processing, stimulation, preservation, fertilization, and insemination, for example, incorporating flow sorting techniques, high purity separation techniques, low dose fertilization and insemination techniques, heterospermic insemination procedures, such as to assess comparative viability, motility, function, or fertility processed in various pressure environments within a sorter, as but a few examples.

The disclosure, such as the various examples provided of separating X chromosome bearing sperm cells from Y chromosome bearing sperm cells, and other disclosed techniques of collection, handling, separation, storage, transportation, usage, fertilization, and insemination are not meant to limit the present invention to any particular embodiment, whether apparatus, method, or otherwise. The descriptions and the various examples should not be construed to limit the present invention to only techniques for sperm sorting or only techniques for sperm preservation. This disclosure, however, may be understood to incorporate the various techniques in the context of the various embodiments of the present invention. Further, the present invention should be considered to incorporate such techniques of sperm processing, preservation, stimulation, fertilization, and insemination consistent with the features disclosed.

As can be easily understood from the foregoing, the basic concepts of the present invention may be embodied in a variety of ways. It involves sperm cell preservation, sperm cell processing, sperm samples, including both techniques as well as devices to accomplish sperm cell processing. In this application, various sperm cell processing techniques are disclosed as part of the results shown to be achieved by the various devices described and as steps which are inherent to utilization. They are simply the natural result of utilizing the devices as intended and described. In addition, while some devices are disclosed, it should be understood that these not only accomplish certain methods but also can be varied in a number of ways. Importantly, as to all of the foregoing, all of these facets should be understood as encompassed by this disclosure.

Further, each of the various elements of the invention and claims may also be achieved in a variety of manners. This disclosure should be understood to encompass each such variation, be it a variation of an embodiment of any apparatus embodiment, a method or process embodiment, or even merely a variation of any element of these. Particularly, it should be understood that as the disclosure relates to elements of the invention, the words for each element may be expressed by equivalent apparatus terms or method terms - even if only the function or result is the same. Such equivalent, broader, or even more generic terms should be considered as encompassed in the description of each element or action. Such terms can be substituted where desired to make explicit the implicitly broad coverage to which this invention is entitled.

As but one example, it should be understood that all actions may be expressed as a means for taking that action or as an element which causes that action. Similarly, each physical element disclosed should be understood to encompass a disclosure of the action which that physical element facilitates. Regarding this last aspect, as but one example, the disclosure of a "cryopreserve element" or "cryopreserve device" or the like should be understood to encompass disclosure of the act of "cryopreserving" -- whether explicitly discussed or not - and, conversely, were there effectively disclosure of the act of "cryopreserving", such a disclosure should be understood to encompass disclosure of a "cryopreserver" and even a "means for cryopreserving." Such changes and alternative terms are to be understood to be explicitly included in the description.

Further, if or when used, the use of the transitional phrase "comprising" is used to maintain the "open-end" claims herein, according to traditional claim interpretation. Thus, unless the context requires otherwise, it should be understood that the term "comprise" or variations such as "comprises" or "comprising", are intended to imply the inclusion of a stated element or step or group of elements or steps but not the exclusion of any other element or step or group of elements or steps. Such terms should be interpreted in their most expansive form so as to afford the applicant the broadest coverage legally permissible.

## Claims

1. A method of mammal sperm cell processing, comprising the steps of:
obtaining mammal sperm celles;
cryopreserving said sperm cells;
thawing said sperm cells;
processing said sperm cells; and
cryopreserving said sperm cells.

2. A method of mammal sperm cell processing as described in claim 1, wherein said step of processing comprises sorting said sperm cells.

3. A method of mammal sperm cell processing as described in claim 2, wherein said step of sorting comprises selecting said sperm cells based upon at least one desired sperm cell characteristic.

4. A method of mammal sperm cell processing as described in claim 3, wherein said step of selecting comprises staining said sperm cells based upon at least one desired sperm cell characteristic.

5. A method of mammal sperm cell processing as described in claim 4, wherein said step of selecting further comprises differentiating said sperm cells based upon said at least one desired sperm cell characteristic.

6. A method of mammal sperm cell processing as described in claim 5, wherein said step of differentiating comprises differentiating said sperm cells as either an X chromosome bearing sperm cell or a Y chromosome bearing sperm cell.

7. A method of mammal sperm cell processing as described in claim 5, wherein said step of selecting further comprises separating said sperm cells having said at least one desired sperm cell characteristic.

8. A method of mammal sperm cell processing as described in claims 5 or 7, further comprising the step of collecting said sperm cells.

9. A method of mammal sperm cell processing as described in claim 2, wherein said step of sorting comprises flow cytometer sorting.

10. A method of mammal sperm cell processing as described in claim 1, further comprising the step of establishing a sperm sample from said processed sperm cells.

11. A method of mammal sperm cell processing as described in claims 10, wherein said second step of cryopreserving comprises cryopreserving said sperm sample.

12. A method of mammal sperm cell processing as described in claim 10, wherein said step of establishing a sperm sample comprises establishing a plurality of sperm samples and wherein said second step of cryopreserving comprises cryopreserving said plurality of samples.

13. A method of mammal sperm cell processing as described in claim 1, further comprising the step of establishing a sperm sample from said processed cryopreserved sperm cells.

14. A method of mammal sperm cell processing as described in claim 1, further comprising the step of thawing said processed cryopreserved sperm cells.

15. A method of mammal sperm cell processing as described in claim 14, further comprising the step of establishing a sperm sample from said processed cryopreserved thawed sperm cells.

16. A method of mammal sperm cell processing as described in claim 13, wherein said step of establishing a sperm sample comprises establishing a plurality of sperm samples.

17. A method of mammal sperm cells processing as described in claim 15, wherein said step of establishing a sperm sample comprising establishing a plurality of sperm samples.

18. A method of mammal sperm cell processing as described in claim 1, wherein said step of obtaining comprises obtaining semen having a concentration of sperm cells, wherein said step of cryopreserving comprises cryopreserving said semen, and wherein said step of thawing comprises thawing said cryopreserved semen.

19. A method of mammal sperm cell processing as described in claim 1, wherein said step of obtaining comprises obtaining sperm cells from zoological specimens.

## Patentansprüche

1. Verfahren zum Verarbeiten von Spermienzellen von Säugern, das die folgenden Schritte aufweist:
Gewinnen von Spermienzellen von Säugern;
Tieftemperaturkonservieren der Spermienzellen;
Auftauen der Spermienzellen;
Verarbeiten der Spermienzellen; und
Tieftemperaturkonservieren der Spermienzellen.

2. Verfahren zum Verarbeiten von Spermienzellen von Säugern nach Anspruch 1, wobei der Schritt des Verarbeitens das Sortieren dieser Spermienzellen umfaßt.

3. Verfahren zum Verarbeiten von Spermienzellen von Säugern nach Anspruch 2, wobei der Schritt des Sortierens das Selektieren dieser Spermienzellen auf der Basis von zumindest einer erwünschten Eigenschaft der Spermienzellen umfaßt.

4. Verfahren zum Verarbeiten von Spermienzellen von Säugern nach Anspruch 3, wobei der Schritt des Selektierens das Färben dieser Spermienzellen auf der Basis von zumindest einer erwünschten Eigenschaft der Spermienzellen umfaßt.

5. Verfahren zum Verarbeiten von Spermienzellen von Säugern nach Anspruch 4, wobei der Schritt des Selektierens ferner das Differenzieren dieser Spermienzellen auf der Basis dieser zumindest einen erwünschten Eigenschaft der Spermienzellen umfaßt.

6. Verfahren zum Verarbeiten von Spermienzellen von Säugern nach Anspruch 5, wobei der Schritt des Differenzierens das Differenzieren dieser Spermienzellen entweder als X-Chromosomen tragende Spermienzelle oder Y-Chromosomen tragende Spermienzelle umfaßt.

7. Verfahren zum Verarbeiten von Spermienzellen von Säugern nach Anspruch 5, wobei der Schritt des Selektierens ferner das Abtrennen dieser Spermienzellen mit dieser zumindest einen erwünschten Eigenschaft der Spermienzellen umfaßt.

8. Verfahren zum Verarbeiten von Spermienzellen von Säugern nach Anspruch 5 oder 7, das ferner den Schritt des Auffangens dieser Spermienzellen umfaßt.

9. Verfahren zum Verarbeiten von Spermienzellen von Säugern nach Anspruch 2, wobei der Schritt des Sortierens das Sortieren mit einem Durchflußzytometer umfaßt.

10. Verfahren zum Verarbeiten von Spermienzellen von Säugern nach Anspruch 1, das ferner den Schritt des Erstellens einer Spermienprobe von diesen verarbeiteten Spermienzellen umfaßt.

11. Verfahren zum Verarbeiten von Spermienzellen von Säugern nach Anspruch 10, wobei der zweite Schritt des Tieftemperaturkonservierens das Tieftemperaturkonservieren dieser Spermienprobe umfaßt.

12. Verfahren zum Verarbeiten von Spermienzellen von Säugern nach Anspruch 10, wobei der Schritt des Erstellens einer Spermienprobe das Erstellen von mehreren Spermienproben umfaßt und wobei der zweite Schritt des Tieftemperaturkonservierens das Tieftemperaturkonservieren dieser mehreren Proben umfaßt.

13. Verfahren zum Verarbeiten von Spermienzellen von Säugern nach Anspruch 1, das ferner den Schritt des Erstellens einer Spermienprobe von diesen verarbeiteten tieftemperaturkonservierten Spermienzellen umfaßt.

14. Verfahren zum Verarbeiten von Spermienzellen von Säugern nach Anspruch 1, das ferner den Schritt des Auftauens dieser verarbeiteten tieftemperaturkonservierten Spermienzellen umfaßt.

15. Verfahren zum Verarbeiten von Spermienzellen von Säugern nach Anspruch 14, das ferner den Schritt des Erstellens einer Spermienprobe von diesen verarbeiteten tieftemperaturkonservierten aufgetauten Spermienzellen umfaßt.

16. Verfahren zum Verarbeiten von Spermienzellen von Säugern nach Anspruch 13, wobei der Schritt des Erstellens einer Spermienprobe das Erstellen von mehreren Spermienproben umfaßt.

17. Verfahren zum Verarbeiten von Spermienzellen von Säugern nach Anspruch 15, wobei der Schritt des Erstellens einer Spermienprobe das Erstellen von mehreren Spermienproben umfaßt.

18. Verfahren zum Verarbeiten von Spermienzellen von Säugern nach Anspruch 1, wobei der Schritt des Gewinnens das Gewinnen von Sperma mit einer Konzentration von Spermienzellen umfaßt, wobei der Schritt des Tieftemperaturkonservierens das Tieftemperaturkonservieren dieses Spermas umfaßt und wobei der Schritt des Auftauens das Auftauen dieses tieftemperaturkonservierten Spermas umfaßt.

19. Verfahren zum Verarbeiten von Spermienzellen von Säugern nach Anspruch 1, wobei der Schritt des Gewinnens das Gewinnen von Spermienzellen von zoologischen Proben umfaßt.

## Revendications

1. Procédé de traitement de cellules spermatiques de mammifères, comprenant les étapes consistant à:
obtenir des cellules spermatiques de mammifères;
cryoconserver lesdites cellules spermatiques;
décongeler lesdites cellules spermatiques;
traiter lesdites cellules spermatiques; et
cryoconserver lesdites cellules spermatiques.

2. Procédé de traitement de cellules spermatiques de mammifères tel que décrit dans la revendication 1, où ladite étape consistant à traiter consiste à trier lesdites cellules spermatiques.

3. Procédé de traitement de cellules spermatiques de mammifères tel que décrit dans la revendication 2, où ladite étape consistant à trier consiste à sélectionner lesdites cellules spermatiques selon au moins une caractéristique de cellules spermatiques désirée.

4. Procédé de traitement de cellules spermatiques de mammifères tel que décrit dans la revendication 3, où ladite étape consistant à sélectionner consiste à colorer lesdites cellules spermatiques selon au moins une caractéristique de cellules spermatiques désirée.

5. Procédé de traitement de cellules spermatiques de mammifères tel que décrit dans la revendication 4, où ladite étape consistant à sélectionner consiste en outre à différencier lesdites cellules spermatiques selon au moins une caractéristique de cellules spermatiques désirée.

6. Procédé de traitement de cellules spermatiques de mammifères tel que décrit dans la revendication 5, où ladite étape consistant à différencier consiste à différencier lesdites cellules spermatiques soit en une cellule spermatique portant un chromosome X ou en une cellule spermatique portant un chromosome Y.

7. Procédé de traitement de cellules spermatiques de mammifères tel que décrit dans la revendication 5, où ladite étape consistant à sélectionner consiste en outre à séparer lesdites cellules spermatiques ayant ladite au moins une caractéristique de cellules spermatiques désirée.

8. Procédé de traitement de cellules spermatiques de mammifères tel que décrit dans les revendications 5 ou 7, comprenant en outre l'étape consistant à recueillir lesdites cellules spermatiques.

9. Procédé de traitement de cellules spermatiques de mammifères tel que décrit dans la revendication 2, où ladite étape consistant à trier consiste à trier par un cytomètre de flux.

10. Procédé de traitement de cellules spermatiques de mammifères tel que décrit dans la revendication 1, comprenant en outre l'étape consistant à établir un échantillon de sperme à partir desdites cellules spermatiques traitées.

11. Procédé de traitement de cellules spermatiques de mammifères tel que décrit dans la revendication 10, où ladite deuxième étape de cryoconservation consiste à cryoconserver ledit échantillon de sperme.

12. Procédé de traitement de cellules spermatiques de mammifères tel que décrit dans la revendication 10, où ladite étape consistant à établir un échantillon de sperme consiste à établir une pluralité d'échantillons de sperme et où ladite deuxième étape de cryoconservation consiste à cryoconserver ladite pluralité d'échantillons.

13. Procédé de traitement de cellules spermatiques de mammifères tel que décrit dans la revendication 1, comprenant en outre l'étape consistant à établir un échantillon de sperme à partir desdites cellules spermatiques cryoconservées traitées.

14. Procédé de traitement de cellules spermatiques de mammifères tel que décrit dans la revendication 1, comprenant en outre l'étape consistant à décongeler lesdites cellules spermatiques cryoconservées traitées.

15. Procédé de traitement de cellules spermatiques de mammifères tel que décrit dans la revendication 14, comprenant en outre l'étape consistant à établir un échantillon de sperme à partir desdites cellules spermatiques décongelées cryoconservées traitées.

16. Procédé de traitement de cellules spermatiques de mammifères tel que décrit dans la revendication 13, où ladite étape consistant à établir un échantillon de sperme consiste à établir une pluralité d'échantillons de sperme.

17. Procédé de traitement de cellules spermatiques de mammifères tel que décrit dans la revendication 15, où ladite étape consistant à établir un échantillon de sperme consiste à établir une pluralité d'échantillons de sperme.

18. Procédé de traitement de cellules spermatiques de mammifères tel que décrit dans la revendication 1, où ladite étape d'obtention consiste à obtenir une semence ayant une concentration de cellules spermatiques, où ladite étape de cryoconservation consiste à cryoconserver ladite semence, et où ladite étape de décongélation consiste à décongeler ladite semence cryoconservée.

19. Procédé de traitement de cellules spermatiques de mammifères tel que décrit dans la revendication 1, où ladite étape d'obtention consiste à obtenir des cellules spermatiques à partir de spécimens zoologiques.
